(19) Europäisches Patentamt
European Patent Office
Office européen des brevets

(11) EP 2 805 722 B1

(12) EUROPEAN PATENT SPECIFICATION

(45) Date of publication and mention of the grant of the patent:
**12.06.2019 Bulletin 2019/24**

(21) Application number: **13738502.7**

(22) Date of filing: **17.01.2013**

(51) Int Cl.:
*A61K 31/7028* (2006.01) *A61K 36/63* (2006.01)
*A23L 2/52* (2006.01) *A23L 33/105* (2016.01)
*A61K 9/16* (2006.01) *A61K 9/20* (2006.01)
*A61K 9/48* (2006.01)

(86) International application number:
**PCT/JP2013/050767**

(87) International publication number:
**WO 2013/108822 (25.07.2013 Gazette 2013/30)**

(54) **DESRHAMNOSYLACTEOSIDE-CONTAINING OLIVE EXTRACT**

DESRHAMNOSYLACTEOSID ENTHALTENDER OLIVENEXTRAKT

EXTRAIT D'OLIVE CONTENANT DU DÉRHAMNOSYL-ACTÉOSIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.01.2012 JP 2012009399**

(43) Date of publication of application:
**26.11.2014 Bulletin 2014/48**

(73) Proprietor: **Suntory Holdings Limited**
**Osaka-shi, Osaka 530-8203 (JP)**

(72) Inventors:
- **FUKUI, Yuko**
**Mishima-gun**
**Osaka 618-8503 (JP)**
- **ONO, Yoshiko**
**Mishima-gun**
**Osaka 618-8503 (JP)**
- **MAEDA, Mitsuru**
**Mishima-gun**
**Osaka 618-0012 (JP)**
- **TOMIMORI, Namino**
**Mishima-gun**
**Osaka 618-8503 (JP)**

(74) Representative: **Vossius & Partner**
**Patentanwälte Rechtsanwälte mbB**
**Siebertstrasse 3**
**81675 München (DE)**

(56) References cited:
**WO-A2-2007/042902** **CN-A- 101 816 665**
**JP-A- H07 206 685** **JP-A- 2001 181 197**

- **AHMAD IJAZ ET AL: "Antioxidant phenylpropanoid glycosides from Buddleja davidii", JOURNAL OF ENZYME INHIBITION AND MEDICINAL CHEMISTRY, vol. 24, no. 4, August 2009 (2009-08), pages 993-997, XP009185484, ISSN: 1475-6366**
- **LIN,L.C. ET AL.: 'Phenylpropanoid glycosides from Orobanche caerulescens' PLANTA MED vol. 70, 2004, pages 50 - 53, XP018001634**
- **AMAKURA,Y. ET AL.: 'Isolation and characterization of phenolic antioxidants from Plantago herb' MOLECULES vol. 17, 09 May 2012, pages 5459 - 5466, XP003031528**
- **HE,J. ET AL.: 'Advanced research on acteoside for chemistry and bioactivities' J ASIAN NAT PROD RES vol. 13, 2011, pages 449 - 464, XP055159937**
- **PURI,M.: 'Updates on naringinase: structural and biotechnological aspects' APPL MICROBIOL BIOTECHNOL vol. 93, 2011, pages 49 - 60, XP019997901**
- **RIBEIRO,M.H.: 'Naringinases: occurrence, characteristics, and applications' APPL MICROBIOL BIOTECHNOL vol. 90, 2011, pages 1883 - 1895, XP019908981**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 805 722 B1

## Description

### TECHNICAL FIELD

The present invention relates to olive extracts comprising desrhamnosyl acteoside (hereinafter referred to as "DRA"), and methods for preparing the same. More particularly, this invention relates to olive extracts comprising DRA and having blood antioxidative activity, and methods for preparing the same.

### BACKGROUND ART

Olive (*Olea europaea*) is a plant belonging to the genus *Olea* of the family *Oleaceae* and is cultivated in wide areas including the Mediterranean region. Olive fruit is used throughout the world for a wide variety of purposes such as for olive oil extraction and for food, and has been eaten by many people for a very long time.

It has been reported that olive fruit contains polyphenols such as oleuropein, hydroxytyrosol, and acteoside (Non-patent Documents 1 and 2), and that olive extracts and their polyphenol compounds have various activities including anti-arteriosclerotic activity (Non-patent Document 3), anti-hypertensive activity (Patent Document 1), bone loss preventing activity (Non-patent Document 4). Olive extracts have also been reported to have antioxidative, whitening, anti-skin aging, and antitumor effects (Patent Document 2).

Acteoside, one of representative polyphenols contained in olive, is known to have antioxidative activity (Non-patent Document 2).

On the other hand, DRA, which has a structure in which a rhamnose unit is removed from acteoside, has been reported to be present in various plants, including *Scrophulariaceae* plants (e.g., *Calceolaria hypericina*) from which DRA is isolated as calceolarioside A (Non-patent Document 5), but DRA has not been found in olive. *In vitro* comparison of antioxidative activities has demonstrated that acteoside is more active than DRA (Non-patent Document 6).

However, investigation of the absorption and excretion of polyphenols, and time-dependent change in their blood antioxidative activities, in humans consuming olive fruits has demonstrated that both parameters were converged in a short time (Non-patent Document 7). Another report has shown that acteoside which is known to have antioxidative activity shows poor absorbability when orally ingested (Non-patent Document 8).

### CITATION LIST

#### PATENT DOCUMENTS

[Patent Document 1] Japanese Patent Application Publication No. JP 2005-334022
[Patent Document 2] Japanese Patent Application Publication No. JP 2002-186453

#### NON-PATENT DOCUMENTS

[Non-patent Document 1] J. Agric Food Chem, vol. 52, pp. 479-484, 2004
[Non-patent Document 2] J. Agric Food Chem, vol. 53, pp. 8963-8969, 2005
[Non-patent Document 3] Atherosclerosis, vol. 188, no. 1, pp. 35-42, 2006
[Non-patent Document 4] Clin Nutr, vol. 25, No. 5, 859-868, 2006
[Non-patent Document 5] Gazzetta Chimica Italiana, 116, pp. 431-433, 1986
[Non-patent Document 6] Planta Medica, 70 (1), 50-53, 2004
[Non-patent Document 7] Phytomedicine, vol. 14, pp. 659-667, 2007
[Non-patent Document 8] J. Chromatogr. B, 844 (1), pp. 89-95, 2006

### SUMMARY OF INVENTION

#### TECHNICAL PROBLEM

As mentioned above, olive fruit contains the antioxidative polyphenol acteoside, but this polyphenol has poor absorbability in the body and does not work well when orally taken. Besides, acteoside can only retain its antioxidative activity for a short duration after ingestion, so in order that acteoside can be used in the form of supplements, it is desired

that acteoside retain its effects longer.

An object of the present invention is to provide foods that are capable of efficiently performing their function in the living body and retaining its blood antioxidative activity for a long time.

SOLUTION TO PROBLEM

In order to solve the above-mentioned problems, the present inventors have made extensive studies and, as a result, surprisingly found that unlike the *in vitro* results, DRA shows higher antioxidative activity in the living body than acteoside and besides that DRA retains its antioxidative activity for a longer duration because it is digested in a different way. The inventors have also found that since DRA is more highly absorbable in the body than acteoside, the DRA metabolites hydroxytyrosol and coffeic acid can perform their various physiological activities efficiently. The inventors have further found that DRA can be prepared by treating acteoside with a glycosidase. Thus, the inventors have completed the present invention, which is as described in the appended claims.

The present disclosure is summarized in the following items (1) to (14):

(1) An olive extract comprising DRA (hereinafter referred to as the "DRA-containing olive extract");
(2) The olive extract according to (1), comprising DRA in an amount of 0.1% by weight or greater;
(3) The olive extract according to (2), comprising DRA in an amount of 1% by weight or greater;
(4) A composition comprising the olive extract according to any one of (1) to (3);
(5) The composition according to (4), wherein the composition is a food or beverage;
(6) A food or beverage comprising DRA in an amount of 0.1% by weight or greater;
(7) The food or beverage according to (6), comprising an olive extract;
(8) A blood antioxidant comprising DRA;
(9) The blood antioxidant according to (8), comprising DRA in an amount of 0.1% by weight or greater;
(10) A composition comprising the blood antioxidant according to (8) or (9);
(11) The composition according to (10), wherein the composition is a food or beverage;
(12) A method for preparing a DRA-containing composition, the method comprising the step of treating an acteoside-containing composition with a glycosidase having rhamnosidase activity;
(13) The method according to (12), wherein the acteoside-containing composition is an olive extract;
(14) The method according to (12) or (13), wherein the glycosidase is naringinase.

ADVANTAGEOUS EFFECTS OF INVENTION

The DRA-containing olive extract of the present invention exhibits high antioxidative activity in the living body for a long time and, thus, is useful as a food or beverage, quasi-drug, or pharmaceutical.

BRIEF DESCRIPTION OF DRAWINGS

FIG. 1 is a graph showing time-dependent change in the amount of DRA produced by enzymatic reaction, with HPLC peak area being used as an index.

FIG. 2 is a graph showing time-dependent change in the amount of acteoside decreasing due to enzymatic reaction, with HPLC peak area being used as an index.

FIG. 3 shows time-dependent change in blood FRAP (Ferric Reducing Ability of Plasma) activity after DRA or acteoside was administered in the form of a suspension in CMC.

FIG. 4 shows time-dependent change in blood FRAP activity after DRA was administered in the form of an olive oil solution.

FIG. 5 shows time-dependent change in blood ORAC (Oxygen Radical Absorbance Capacity) activity after DRA or acteoside was administered in the form of a suspension in CMC.

FIG. 6 shows time-dependent change in blood ORAC activity after DRA was administered in the form of an olive oil solution.

FIG. 7 is a drawing showing time-dependent change in the concentrations of acteoside, DRA, hydroxytyrosol and coffeic acid in the blood of rats administered with a suspension in 0.5% CMC.

FIG. 8 is a drawing showing time-dependent change in the concentrations of acteoside, DRA, hydroxytyrosol and coffeic acid in the blood of rats administered with a suspension of 500 mg acteoside in 0.5% CMC.

FIG. 9 is a drawing showing time-dependent change in the concentrations of hydroxytyrosol and coffeic acid in the

blood of rats administered with a suspension of 423 mg DRA in 0.5% CMC.

FIG. 10 is a drawing showing that the amount of DRA absorbed in the body (AUC) is greater than that of acteoside.

FIG. 11 shows time-dependent change in blood FRAP activity after solutions of different concentrations of DRA-containing extracts dissolved in olive oil were each administered in a single dose.

FIG. 12 shows time-dependent change in blood FRAP activity after a suspension of a DRA-containing olive extract or a non-enzymatically treated olive extract in olive oil was administered in a single dose.

FIG. 13 shows the blood FRAP activity at 9 hours after the final administration of consecutive doses of a DRA-containing olive extract or a non-enzymatically treated olive extract in the form of a suspension in olive oil.

DESCRIPTION OF EMBODIMENTS

The present invention relates to an olive extract comprising DRA in an amount of 0.1% by weight or greater and exhibiting high antioxidative activity in the living body for a long time, a food or beverage comprising the olive extract, and a method for preparing the same.

On the pages that follow, embodiments of the present invention will be described in more detail.

<DRA-containing olive extract>

The DRA-containing olive extract of the present invention comprises DRA. The structural formula of DRA is shown below.

[Formula 1]

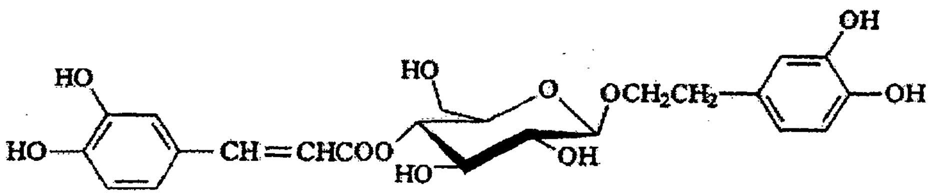

As mentioned above, DRA was first isolated as calceolarioside A from *Scrophulariaceae* plants (e.g., *Calceolaria hypericina*) in 1986, and since then has been reported to be present in various plants but has not been found in olive. On the other hand, the DRA-containing olive extract of the present invention may contain even only in a very small amount, and contains it in an amount of 0.1% by weight or greater, preferably 1% by weight or greater, and more preferably 5% by weight or greater.

DRA may be obtained by enzymatically treating acteoside shown below or by any other method. For example, a DRA-containing olive extract can be obtained by reacting an acteoside-containing olive extract under appropriate conditions with an enzyme capable of removing a rhamnose moiety from acteoside, or by extracting DRA from a DRA-containing plant material or the like and adding it to an olive extract.

The structural formula of acteoside is shown below.

[Formula 2]

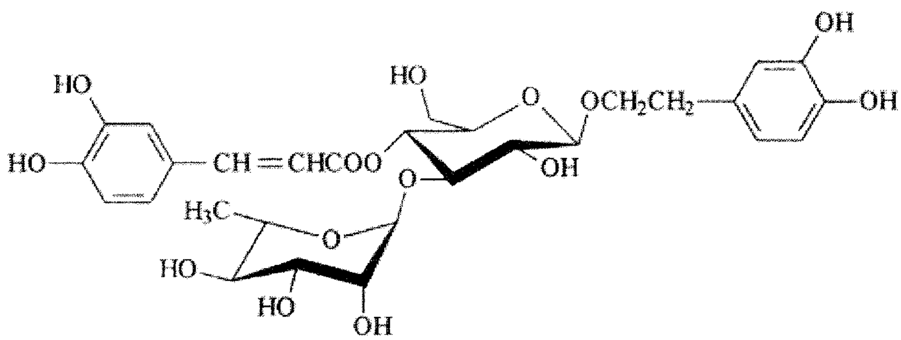

It is sufficient that the DRA be contained in DRA-containing olive extract of the present invention, and any other components can be contained in this olive extract without particular limitation. Examples of other components that can be incorporated include olive-derived components such as acteoside and hydroxytyrosol. The contents of such other

components and the relative ratio of DRA to such other components can be set as desired.

Olive is a plant belonging to the genus *Olea* of the family *Oleaceae*, and any varieties of olive can be used as a source material for extract preparation. Examples of olive varieties that can advantageously be used include Manzanillo, Lucca, Nevadillo Blanco, Mission, Picual, Arbechina, Hojiblanca, Cornicabra, Gordal, Moraiolo, Frantoio, Coratina, and Leccino.

For olive extract preparation, any parts of the plant, such as fruit, seed, leaf, and stem, can be used, but those parts with high acteoside content, such as fruit, are preferably used. Olive fruit can be used raw or can be dried as by freeze-drying before use. Also, residues remaining after oil is squeezed from olive fruits can be used directly or in a dried state. The olive extract preparation is achieved by subjecting any of the above-mentioned source materials to solvent extraction. The solvent used for the solvent extraction can be a polar or nonpolar solvent. Examples of the solvent include water, alcohols such as methanol or ethanol, polyhydric alcohols such as ethylene glycol or propylene glycol, and ketones, with hot water at 60-90°C being preferred.

The DRA-containing olive extract of the present invention has various effects due to its containing DRA. For example, since DRA is more highly absorbable in the body than acteoside, the DRA metabolites hydroxytyrosol and coffeic acid can perform their various physiological activities more efficiently. Further, DRA retains its blood antioxidative activity for a long duration after ingestion.

The amount of DRA or the like absorbed in the body can be determined by, for example, administering a test solution to a test animal, collecting a blood sample from the animal after a certain period of time, and performing concentration measurement on the sample. By way of example, a rat fasted overnight is orally administered using a sonde with a suspension of DRA in olive oil (0.8 mM) at a dose of 5 mL/kg. At regular intervals after the administration, a blood sample is collected from the caudal vein of the rat into a heparin blood tube and is centrifuged to obtain a plasma sample, and concentration analysis is conducted to thereby determine the DRA amount.

The structural formulas of the DRA metabolites hydroxytyrosol and coffeic acid are shown below.

[Formula 3]

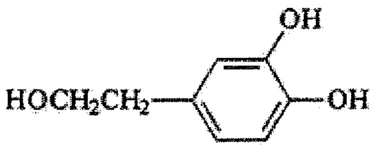

Hydroxytyrosol

[Formula 4]

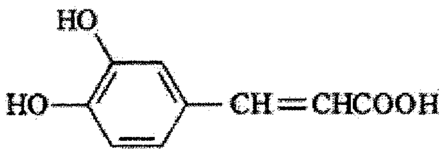

Coffeic acid

Hydroxytyrosol is known to have very strong antioxidative activity and to be capable of preventing the bad cholesterol, LDL, from turning into the worse cholesterol, oxidized LDL.

Coffeic acid is known as one of polyphenols contained in coffee and as an aroma component. It has been reported that coffeic acid is effective in suppressing the growth and metastasis of cancer cells.

Olive extracts are widely used as foods throughout the world and have been eaten by many people for a very long time. Thus, the DRA-containing olive extract of the present invention can be used directly as a food, beverage or the like, or can be incorporated in a food, beverage, pharmaceutical or the like.

In cases where the DRA-containing olive extract is used directly as a food, beverage or the like, other physiologically active components, including minerals, vitamins such as vitamin E, vitamin C and vitamin A, nutritional components, flavorings, pigments, and other additives, can be added depending on the need, as long as they do not impair the effects of DRA or, in other words, do not unfavorably interact with DRA. All of the additives that can be used are those commonly used in foods and beverages.

The DRA-containing olive extract can also be formulated into functional foods (including health foods such as health supplementary foods, nutritional functional foods, foods for special dietary use, and foods for special health use,

as well as animal supplements), animal feeds, and others.

<Foods and beverages>

The present invention relates to foods and beverages comprising the olive extract according to the invention.

DRA may be obtained by enzymatically treating acteoside or by any other method. For example, a DRA-containing product can be obtained by reacting an acteoside-containing product under appropriate conditions with an enzyme capable of removing a rhamnose moiety from acteoside, or by extracting DRA from a DRA-containing plant material or the like.

The inventive food or beverage can be provided in the form of health foods such as tablets, capsules, powders, granules and drinkable preparations (including solutions and suspensions) or in the form of refreshing drinks, tea beverages, dairy products such as yoghurt drinks and lactic fermenting drinks, seasonings, processed foods, desserts, confectioneries (e.g., gum, candy, jelly), and the like. The food or beverage of the present invention may contain excipients, binding agents, coating agents, disintegrating agents, and/or other additives that are acceptable as foods ingredients.

Because of having antioxidative activity, the food or beverage of the present invention is effective in preventing or ameliorating disorders, diseases, etc. associated with reactive oxygen species.

<Blood antioxidants>

Disclosed herein are DRA-containing blood antioxidants.

As mentioned above, it has been found that unlike previous reports, there occurs a significant increase in the antioxidative activity in the blood after DRA administration, whereas little increase in the blood antioxidative activity occurs after acteoside administration. Besides, DRA retains its effects for a long duration; so the DRA-containing blood antioxidant is a sustained-release blood antioxidant. Because of having antioxidative activity, the blood antioxidant is effective in preventing or ameliorating disorders, diseases, etc. associated with reactive oxygen species.

Blood antioxidative activity can be determined by, for example, using the FRAP (Ferric Reducing Ability of Plasma) or ORAC (Oxygen Radical Absorbance Capacity) method.

The amount of DRA present in the blood antioxidant is not particularly limited but from the viewpoint of its effects, this amount is 0.1% by weight or greater, preferably 1% by weight or greater, more preferably 2% by weight or greater, and still more preferably 5% by weight or greater. DRA may be obtained by enzymatically treating acteoside or by any other method. For example, a DRA-containing product can be obtained by reacting an acteoside-containing product under appropriate conditions with an enzyme capable of removing a rhamnose moiety from acteoside, or by extracting DRA from a DRA-containing plant material or the like.

The blood antioxidant can be provided in oral dosage forms such as tablets, capsules, granules, powders, and syrups or in non-oral dosage forms such as injections. The blood antioxidant may contain pharmaceutically acceptable excipients, binding agents, bulking agents, dispersing agents, preservatives, or other additives.

The blood antioxidant can be used directly or incorporated in a food, beverage, pharmaceutical or the like.

In cases where the blood antioxidant is used directly as a food, beverage or the like, other physiologically active components, including minerals, vitamins such as vitamin E, vitamin C and vitamin A, nutritional components, flavorings, pigments, and other additives, can be added depending on the need, as long as they do not impair the effects of DRA or, in other words, do not unfavorably interact with DRA. All of the additives that can be used are those commonly used in foods and beverages.

<Methods for preparing a DRA-containing composition>

The preparation method of the present invention prepares a DRA-containing composition from an acteoside-containing composition. In particular, a DRA-containing composition can be simply prepared by removing a rhamnose moiety from acteoside through glycosidase treatment.

The acteoside-containing composition to be used as a source material can be of any type as long as it contains acteoside. It can be purified acteoside or a mixture of acteoside with any other polyphenolic compound such as oleuropein or hydroxytyrosol. Acteoside-containing plant extracts can also be used. The concentration of acteoside in a source material can be decided as appropriate in consideration of the amount of DRA to be included in the intended product. As mentioned above, olive contains acteoside in a relatively large amount, and an olive extract containing acteoside in a desired proportion can be obtained from olive using a known method as appropriate; thus, olive is a preferred source material in the preparation method of the present invention. In this respect, olive fruit is preferred, or alternatively a solution of a commercially available olive fruit extract powder can be used.

Any glycosidase can be used without limitation as long as it has rhamnosidase activity for removing rhamnose from acteoside. Exemplary enzymes having rhamnosidase activity include naringinase and hesperidinase. Enzymatic

treatment conditions can be decided as appropriate in consideration of various factors including pH and the optimum reaction temperature of the enzyme to be used. For example, in the case of using naringinase, reaction is effected at a temperature ranging from 30 to 60°C, preferably from 35 to 45°C, with the pH adjusted within the range from 3.5 to 5 using an appropriate reagent. After completion of the enzymatic treatment, the enzyme is deactivated by using a known appropriate method, for example, by adjusting the solution pH to 3 and performing heating at 80°C or higher for 10 minutes. The desired DRA concentration in a produced DRA-containing composition can be obtained by appropriately setting the acteoside concentration in a source material, the amount of the enzyme to be added, reaction time, and the like.

The concentrations of DRA and residual acteoside in a DRA-containing composition obtained by enzymatic reaction can be determined by a method known to those skilled in the art, such as HPLC analysis.

It is also possible to use DRA separated and purified from a prepared DRA-containing composition. DRA separation and purification can be performed as appropriate by a method known to those skilled in the art, such as solvent extraction or chromatographic separation.

## EXAMPLES

On the pages that follow, the present invention will be described by way of Examples, but this invention is not limited to these examples.

### Preparation Example 1 Preparation of An Olive Extract

Eight kilograms of residues remaining after oil had been squeezed from olive fruits were subjected to extraction twice, each time for 30 minutes, using 32 L of hot water at 80°C. After filtration was performed using a nylon mesh (produced by Nippon Rikagaku Kikai Co., Ltd.; product name: NRS-500), the residues were removed by suction filtration using two sheets of # 131 (ϕ330 mm) filter papers and 200 g of Hyflo Super-Cel (Nacalai Tesque. Inc.) to thereby obtain an extract. The entire amount of the extract was loaded on 5 L of the Amberlite XAD7-HP resin (Rohm and Haas Japan K.K.) (column size: ϕ14×32 cm) that had been washed with 50% acetone and equilibrated with water. After washing with 5 L of water, elution was sequentially performed with 20 L of water, 35 L of a 15% ethanol solution, and 20 L of a 60% ethanol solution. The 15% ethanol-eluted fraction and 60% ethanol-eluted fraction were each concentrated under reduced pressure and freeze-dried to obtain 37.2 g and 66.0 g of fractionated products, respectively. Of the two fractionated products, the one obtained from the 60% ethanol-eluted fraction was used in subsequent animal tests as an olive extract (acteoside content: 9.1%).

### Preparation Example 2 Preparation of Acteoside

One hundred grams of an extract powder from olive pulps (Oleaselect™ produced by Indena (Batch No. 10219)) was dissolved in 1200 mL of methyl ethyl ketone and 600 mL of distilled water, and the solution was shaken well in a 3 L volume separating funnel to recover an organic layer. 1200 mL of methyl ethyl ketone was added to an aqueous layer, and the solution was shaken well to recover an organic layer, and the same procedure was repeated once more. The organic layers obtained by a total of three rounds of separation were mixed, concentrated and freeze-dried to obtain 52.5 g of a dry product.

The entire amount of the dry product was dissolved in 200 mL of a 70% aqueous ethanol solution and further diluted 10-fold with water, and then loaded on 1 L of MCI GEL CHP-20P (produced by Mitsubishi Chemical Corporation; 75-150 μm) which had been equilibrated with water. After 10 L of a 10% aqueous ethanol solution was passed, elution was sequentially performed with 1 L each of 12.5%, 15%, 17.5%, 20%, 22.5%, 25%, 27.5%, 30% and 35% aqueous ethanol solutions and 2 L of a 40% aqueous ethanol solution (all ethanol concentrations are given in %V/V). The 12.5% to 27.5% eluates were fractionated into 4 fractions of 250 mL each and, of these fractions, the fourth one of the 20% eluate up to the third one of the 25% eluate were mixed and concentrated to obtain 5.98 g of purified acteoside.

### Example 1 Preparation of DRAs -- 1

First, 0.37 mg of naringinase (derived from *Penicillium decumbens*; 300 units/g; produced by Sigma) was suspended in 1.68 mL of a 0.1 M acetate buffer (pH 4.0) to prepare a 0.22 mg/mL enzyme solution (abbreviated as 1/10). The 1/10 enzyme solution was diluted with a 0.1 M acetate buffer (pH 4.0) to prepare enzyme solutions diluted 3-, 10- and 30-fold (abbreviated as 1/30, 1/100 and 1/300, respectively). 0.9 mL each of the enzyme solutions was taken in a screw-cap glass test tube, 0.1 mL of a 50% aqueous ethanol solution (20 mg/mL) of the purified acteoside obtained in Preparation Example 2 was added, and then the tube was shaken at 40°C (at 100-130 cycles/min. using the MM-10 Water Bath Shaker produced by Taiyo Kagaku Kogyo Co., Ltd. (now Tietech Co., Ltd.). 1, 2, 3, 4, 5 and 24 hours after the initiation of the reaction, 0.1 mL of the sample was taken, and for the purpose of deproteinization, an equivalent

amount of ice-cold acetonitrile was added and the mixture was stirred, left to stand on ice for 10 minutes and centrifuged (at 10,000 rotations for 10 min. at 5°C using the MX-100 high-speed refrigerated centrifuge produced by Tomy Seiko Co., Ltd.). 4 μL each of the suspensions obtained after the centrifugation was subjected to HPLC analysis under the following conditions. The samples taken from an enzyme-free control (abbreviated as Ez(-)) at 5 and 24 hours after administration were treated in the same way to check the stability of acteoside. Also, LC/MS analysis was conducted to confirm that DRA was produced by the reaction.

<HPLC analysis conditions>

System: Waters 2690/2695 Separations Modules and 996 Photodiode Array Detector
Column: Develosil™ RPAQUEOUS-AR-5 (3×150 mm)
Mobile phase: Solution A: 0.1% formic acid- 15% acetonitrile/distilled water; Solution B: 0.1% formic acid-50% acetonitrile/distilled water
Program: 0 min. (0% Solution B) and 30 min. (100% Solution B)
Flow rate: 0.43 mL/min.
Injection volume: 4 μL
Detection wavelength: 280 nm

Under the above-mentioned analysis conditions, DRA was eluted at 8.958±0.008 minutes (n=24) and acteoside at 9.348±0.009 minutes (n=18). The results of time-dependent change in HPLC peak area as an index are shown in FIGs. 1 and 2. It was confirmed that as the amount of acteoside decreases, DRA is produced in a greater amount.

Example 2 Preparation of DRA -- 2

Four grams of the purified acteoside obtained in Preparation Example 2 was dissolved in 100 mL of a 50% aqueous ethanol solution. The entire amount of the resulting solution was added to 2 L of a 0.1 M acetate buffer (pH 4.0), the mixture was kept at 40°C, and 400 mg of naringinase (derived from *Penicillium decumbens*; 300 units/g; produced by Sigma) was added. Immediately after stirring at 40°C for 3 hours, the entire amount of the reaction liquid was loaded on 700 mL of MCI GEL CHP-20P (produced by Mitsubishi Chemical Corporation; 75-150 μm) which had been equilibrated with water. After washing with 2 L of water, 2 L of a 15% aqueous ethanol solution and 1200 mL of a 20% aqueous ethanol solution were passed. Then, elution was performed with 750 mL each of 22.5%, 25% and 27.5% aqueous ethanol solutions, and each of the eluates was fractionated into three fractions of 250 mL each. Further elution was carried out with 1250 mL of a 30% aqueous ethanol solution, and the eluate was fractionated into five fractions of 250 mL each. Of these fractions, a mixture of the first and second fractions of the 27.5% eluate and a mixture of the third fraction of the 27.5% eluate to the third fraction of the 30% eluate were concentrated separately to obtain 438 mg and 1.562 g of dry products, respectively. MS and NMR analyses were conducted on the dry product of the mixture of the third fraction of the 27.5% eluate to the third fraction of the 30% eluate to confirm that DRA was produced by the reaction.

Example 3 Preparation of A DRA-Containing Olive Extract -- 1

One hundred and sixty milliliters of distilled water was incubated at 40°C, 20 mL of a 50% aqueous ethanol solution of 4 g of an extract powder from olive pulps (Oleaselect™ produced by Indena (Batch No. 10219)) was added, and the mixture was stirred (the pH at this stage was pH 4.7); then, the pH of the mixture was adjusted to 4.0 with glacial acetic acid. Next, 20 mL of a suspension of 40 mg naringinase (derived from *Penicillium decumbens*; 300 units/g; produced by Sigma) in distilled water was added to initiate the reaction. At 0.5 minute, at 2-minute intervals from 2 to 30 minutes, and at 30-minute intervals from 60 to 210 minutes, 100 μL of the sample was taken, and for the purpose of deproteinization, an equivalent amount (100 μL) of ice-cold acetonitrile was added and the mixture was stirred and centrifuged (at 10,000 rotations for 10 min. at 5°C using the MX-100 high-speed refrigerated centrifuge produced by Tomy Seiko Co., Ltd.). After warming for 210 minutes, the pH of the reaction liquid was 4.1. Also, a sample obtained after adding distilled water in place of an enzyme solution and treating the mixture in the same manner was used as a control. 4 μL each of the suspensions obtained after the centrifugation was subjected to HPLC analysis to determine the peak areas of DRA and acteoside. Further, liquids prepared by stepwise diluting each of DRA and acteoside with 50% acetonitrile were analyzed under the same conditions to construct calibration curves ($R^2$ = 0.9999 for the DRA calibration curve, and $R^2$ = 1 for the acteoside calibration curve). The DRA and acteoside samples used for constructing the calibration curves were those obtained in Example 2 and Preparation Example 2, respectively. Concentrations were

calculated from the peak area values and the calibration curves to determine the contents of DRA and acteoside in the original samples (the concentration upon analysis was equivalent to 10 mg/mL) according to the following calculation equation. The results are shown in Table 1.

<HPLC analysis conditions>

System: Waters 2690/2695 Separations Modules and 996 Photodiode Array Detector
Column: Develosil™ RPAQUEOUS-AR-5 (3×150 mm)
Mobile phase: Solution A: 0. 1% formic acid-15% acetonitrile/distilled water; Solution B: 0.1% formic acid-60% acetonitrile/distilled water
Program: 0 min. (0% Solution B) and 30 min. (100% Solution B)
Flow rate: 0.43 mL/min.
Injection volume: 4 μL,
Detection wavelength: 280 nm

<Calculation equation>

The sample concentration upon reaction was 20 mg/mL, and the sample concentration upon analysis was equivalent to 10 mg/mL because the sample was diluted 2-fold with acetonitrile, and therefore the calculation equation is expressed as follows:

$$\text{Content (\%)} = 100 \times \text{Concentration } (\mu g/mL) / 10000 \ (\mu g/mL).$$

Table 1 shows the time-dependent changes in DRA and acteoside contents. It was confirmed that as the amount of acteoside decreases, DRA is produced in a greater amount.

[Table 1]

| | Content (%) | |
|---|---|---|
| Reaction time (min.) | Desrhamosyl acteoside | Acteoside |
| 0.5 | 0.0 | 10.6 |
| 2 | 0.1 | 10.5 |
| 4 | 0.3 | 9.9 |
| 6 | 0.5 | 9.9 |
| 8 | 0.7 | 9.6 |
| 10 | 0.8 | 9.3 |
| 12 | 1.0 | 9.4 |
| 14 | 1.2 | 9.1 |
| 16 | 1.4 | 8.9 |
| 18 | 1.6 | 8.9 |
| 20 | 1.7 | 8.6 |
| 22 | 1.9 | 8.5 |
| 24 | 2.0 | 8.1 |
| 26 | 2.3 | 8.5 |
| 28 | 2.4 | 8.1 |
| 30 | 2.8 | 8.2 |
| 60 | 4.9 | 5.3 |

(continued)

| | Content (%) | |
|---|---|---|
| Reaction time (min.) | Desrhamosyl acteoside | Acteoside |
| 90 | 6.4 | 2.9 |
| 120 | 7.5 | 1.5 |
| 150 | 8.1 | 0.8 |
| 180 | 8.1 | 0.4 |
| 210 | 8.4 | 0.0 |
| 0 (Control) | | 10.6 |

Example 4 Preparation of A DRA-Containing Olive Extract -- 2

<Extraction>

Two kilograms of residues remaining after oil had been squeezed from olive fruits were subjected to extraction using 16 L of hot water at 80°C for 2 hours. After filtration was performed using a nylon mesh (produced by Nippon Rikagaku Kikai Co., Ltd.; product name: NRS-500), the residues were removed by suction filtration using two sheets of #13 1 (ϕ330 mm) filter papers and 400 g of Hyflo Super-Cel (Nacalai Tesque, Inc.) to thereby obtain an extract.

<Desrhamnosyl reaction>

The extract was cooled to 40°C, and 32 mL of acetic acid was added to reduce the pH to 3.93. While the temperature was kept at 40°C in a water bath, 130 mg of naringinase (derived from *Penicillium decumbens*; 540 units/g; produced by Sigma) was added, and reaction was effected for 3.5 hours. In order to terminate the reaction, the enzyme was deactivated by adding 35 mL of 6 N sulfuric acid to adjust the pH to 3.0 and performing heating at 80°C for 10 minutes. Then, the reaction liquid was cooled to 40°C or lower in a water bath, and an equivalent amount (52.5 mL) of a 4 N aqueous NaOH solution was added.

<Purification>

The entire amount of the foregoing reaction liquid was loaded on 1 L of the Amberlite XAD7-HP resin (Rohm and Haas Japan K.K.) (column size: ϕ8×20 cm) that had been washed with 50% acetone and equilibrated with water. After washing with 2 L of water, elution was sequentially performed with 6 L of a 15% ethanol solution and 4 L of a 60% ethanol solution. The 15% ethanol-eluted fraction and 60% ethanol-eluted fraction were each concentrated under reduced pressure and freeze-dried to obtain 8.73 g and 16.8 g of fractionated products, respectively. Of the two fractionated products, the one obtained from the 60% ethanol-eluted fraction was used in subsequent animal tests as a DRA-containing olive extract (DRA content: 8.9%).

Example 5 Blood Antioxidative Activity of DRA

<Determination method>

The influence of DRA on blood antioxidative activity was evaluated using rats. The evaluation of blood antioxidative activity was performed using the FRAP and ORAC methods. The DRA and acteoside used in this evaluation were those prepared by the same procedures as in Example 2 and Preparation Example 2, respectively.

SD (IGS) male rats (6 weeks old) were purchased from Charles River Laboratories Japan, Inc. After the rats were acclimated to the test environment for one week, those rats which grew normally were subjected to testing. The rats were fasted overnight and divided into five groups each consisting of three rats. Rats were orally administered a 0.5% CMC solution (Group 1), a suspension of DRA in 0.5% CMC (0.8 mM) (Group 2), a suspension of purified acteoside in 0.5% CMC (0.8 mM) (Group 3), an olive oil solution (Nacalai Tesque, Inc.; Code No. 073-26) (Group 4), and a suspension of DRA in olive oil (0.8 mM) (Group 5), each at a dose of 5 mL/kg. Before administration and 0.5, 1, 3, 6, 9 and 24 hours after administration for Groups 1-3, or before administration and 1, 3, 6, 9 and 24 hours after administration for Groups 4-5, a blood sample was collected from the caudal vein in heparinized tube and was centrifuged (at 8,000

rpm for 10 min.) to obtain plasma sample. At a later date, plasma FRAP activity as well as the ORAC activity of the supernatants obtained after deproteinization with acetone were evaluated.

The results are shown in FIGs. 3-6. The acteoside group showed little increase in blood antioxidative activity while the DRA groups showed an apparent increase in blood antioxidative activity after administration. Particularly from the viewpoint of FRAP activity, the antioxidative activity showed bimodal peaks at an earlier time (1-3 hours) and at 9 hours after administration; thus, it was confirmed that DRA has a sustained-release property.

Example 6 Absorption in the Body

Among the plasma samples collected for the determination of blood antioxidative activity in Example 5, some parts of those samples from Group 1 (a 0.5% CMC solution), Group 2 (a suspension of DRA in 0.5% CMC) and Group 3 (a suspension of acteoside in 0.5% CMC) were used to determine the blood concentrations of the respective samples after administration.

<Determination method>

After equivalent amounts of the plasma samples from the three rats in each group were mixed and homogenized, 90 μL of a *helix pomatia*-derived β-glucuronidase/arylsulphatase/acetate buffer (pH 5.0) was added to 90 μL of the plasma mixture and incubation was conducted at 37°C for one hour. After 900 μL of acetonitrile was added to terminate the reaction, 10 μL of an aqueous solution of 1% ascorbic acid and 10 μL of an internal standard solution were added, and the contents were mixed and centrifuged (at 15,000 rpm for 10 min.) to recover a supernatant. The recovered supernatant was concentrated under reduced pressure, dissolved again in 50% methanol, passed through a filter, and subjected to LC-MS/MS analysis to quantify acteoside, DRA, hydroxytyrosol, and coffeic acid. The amounts of acteoside, DRA, hydroxytyrosol, and coffeic acid were determined by the ratio of each of their peak areas to the peak area of hesperidin (Wako) used as an internal standard. The LC-MS/MS analysis conditions are shown below.

<HPLC analysis conditions>

Column: ACQUITY BEH18 (1.7 μm, 2.1Φ×100 mm; Waters Japan Inc.)
Mobile phase: Solution A: 0.1% aqueous formic acid solution; Solution B: acetonitrile
Flow rate: 0.30 mL/min.
Gradient: 5% Solution B for 5 minutes; from 5% to 10% Solution B over 5 minutes; 10% Solution B for 2 minutes; from 10% to 24% Solution B over 7 minutes; and a linear gradient from 24% to 80% Solution B for 4 minutes

<MS/MS analysis conditions>

Determination mode: Selected reaction monitoring
Detection:

Acteoside (retention time: about 17.4 min.); precursor ion at *m*/*z* = 623 ($[M-H]^-$), product ion at *m*/*z* = 161
DRA (retention time: about 17.0 min.); precursor ion at *m*/*z* = 477 ($[M-H]^-$), product ion at *m*/*z* = 161
Hydroxytyrosol (retention time: about 3.1 min.); precursor ion at *m*/*z* = 153 ($[M-H]^-$), product ion at *m*/*z* = 123
Coffeic acid (retention time: about 8.1 min.); precursor ion at *m*/*z* = 179 ($[M-H]^-$), product ion at *m*/*z* = 135
Hesperidin (retention time: about 18.7 min.); precursor ion at *m*/*z* = 609 ($[M-H]^-$), product ion at *m*/*z* = 301

Ionization method: ESI

The results are shown in FIGs. 7, 8 and 9. In the case of acteoside administration, the maximum blood concentration ($C_{max}$) of acteoside did not reach even 1 μM, but its metabolites hydroxytyrosol and coffeic acid were detected. The $C_{max}$s of hydroxytyrosol and coffeic acid were 11.7 μM and 0.7 μM, respectively. On the other hand, in the case of DRA administration, the $C_{max}$ of DRA did also not reach even 1 μM, but its metabolites hydroxytyrosol and coffeic acid were detected at high concentrations. The $C_{max}$s of hydroxytyrosol and coffeic acid were 32.8 μM and 7.3 μM, respectively.

Both acteoside and DRA were not absorbed or transferred into the blood as they were, and they were present

in the body in the form of their metabolites hydroxytyrosol and coffeic acid. Thus, the amounts of acteoside and DRA absorbed in the body were compared using the AUCs of their hydroxytyrosols and coffeic acids, and the results of this comparison are shown in FIG. 10. The amount of DRA absorbed in the body was about 3 times greater than that of acteoside according to the comparison between the AUCs of their hydroxytyrosols, or was about 13 times greater than according to the comparison between the AUCs of their coffeic acids.

The above-mentioned results suggest that as compared with acteoside, DRA is absorbed in the body in greater amounts and its metabolite hydroxytyrosol and coffeic acid show various physiological activities at higher levels.

Example 7 Evaluation of the Dose Dependency of Blood Antioxidative Activity

The blood antioxidative activities of olive extracts containing different concentrations of DRA were compared.

determination method>

SD (IGS) male rats (6 weeks old) were purchased from Charles River Laboratories Japan, Inc. After the rats were acclimated to the test environment for one week, those rats which grew normally were fasted overnight, and they were divided into five groups each consisting of four rats. A DRA-containing olive extract was prepared using DRA prepared by the same procedure as in Example 2 and the olive extract obtained in Preparation Example 1. The DRA-containing olive extract was dissolved in an olive oil to prepare solutions for administration each containing DRA at concentrations of 0, 0.5, 1, 2 or 5%. The solutions were orally administered each at a dose of 5 mL/kg. Before administration and 1, 3, 6, 9 and 24 hours after administration, a blood sample was collected from the caudal vein into a heparinized tube and was centrifuged (at 8,000 rpm for 10 min.) to obtain a plasma sample. At a later date, plasma FRAP activity was determined.

The results are shown in FIG. 11. The blood antioxidative activity increased in a DRA dose-dependent manner, and an apparent elevating effect on blood antioxidative activity was observed at DRA concentrations of 2% or higher.

Example 8 Blood Antioxidative Activity

The blood antioxidative activities of olive extracts which had or had not undergone desrhamnosyl reaction were compared in terms of FRAP activity.

<Determination method>

The samples used in this determination were the DRA-containing olive extract (DRA content: 8.9%) prepared in Example 4 and a non-enzymatically treated olive extract (acteoside content: 9.1%) obtained by the same treatment as in Example 4 except that no desrhamnosyl reaction was effected.

SD (IGS) male rats (6 weeks old) were purchased from Charles River Laboratories Japan, Inc. After the rats were acclimated to the test environment for one week, those rats which grew normally were subjected to testing. The rats fasted overnight were divided into three groups each consisting of four rats. The rats were orally administered an olive oil solution (Group 1), a suspension of the DRA-containing olive extract in olive oil (Group 2), and a suspension of the non-enzymatically treated olive extract in olive oil (Group 3), each at a dose of 500 mg/5 mL/kg. Before administration and 1, 3, 6, 9 and 24 hours after administration, a blood sample was collected from the caudal vein into a heparinized tube and was centrifuged (at 8,000 rpm for 10 min.) to obtain a plasma sample. At a later date, plasma FRAP activity was determined.

The results are shown in FIG. 12. It was confirmed that the antioxidative activity is elevated by increasing the DRA content by enzymatic treatment.

Example 9 Sustained-Release Property of DRA

Test animals and solutions for administration were provided by the same procedures as in Example 8. Administration was repeated every 24 hours for 5 consecutive days at a dose of 500 mg/5 mL/kg, and 9 hours after the final administration, a blood sample was collected in a heparinized tube and was centrifuged (at 8,000 rpm for 10 min.) to obtain a plasma sample. At a later date, plasma FRAP activity was determined.

The results are shown in FIG. 13. As compared with the non-enzymatically treated olive extract group, the DRA-containing olive extract group showed a significant increase in blood antioxidative activity at 9 hours after the final administration; thus, it was confirmed that DRA has a sustained-release property.

Formulation Examples 1 Tablet

| | (Amount per tablet) |
|---|---|
| DRA-containing olive extract (DRA content: 8.9%) | 25 mg |
| Silicic anhydride | 5 mg |
| Microcrystalline cellulose | 120 mg |
| Maltitol | 150 mg |

The above-mentioned components were uniformly mixed and tabletted in a single punch tabletting machine to prepare a tablet having a diameter of 10 mm and weighing 300 mg. The recommended dose is 4 tablets per day.

Formulation Example 2 Granules

| | (Amount per stick) |
|---|---|
| DRA-containing olive extract (DRA content: 8.9%) | 150 mg |
| Corn starch | 400 mg |
| Maltitol | 1,000 mg |

After the above-mentioned components were uniformly mixed, 100 mL of a 10% hydroxypropyl cellulose/ethanol solution was added, and the mixture was kneaded to homogenize, extruded and dried according to conventional methods to obtain granules. The daily dose is one stick containing 1.5 g of granules.

Formulation Example 3 Soft Capsule

The relative amounts per capsule are shown below. The recommended dose is 2 capsules per day.

| | (% by weight) |
|---|---|
| Gelatin | 70.0 |
| Glycerin | 29.7 |
| Caramel | 0.3 |
| Water | Proper amount |
| Total | 100 |

A soft capsule shell composed of the above-mentioned components was filled with the composition mentioned below according to a conventional method to obtain a 300 mg soft capsule. The recommended dose of this soft capsule is 2 capsules per day.

| | (Amount per capsule) |
|---|---|
| DRA-containing olive extract (DRA content: 8.9%) | 50 mg |
| Beeswax | 0.02 mL |
| Olive oil | 0.1 mL |

Formulation Example 4 Drinkable Preparation

| | | |
|---|---|---|
| Savoring: | Disodium DL-tartrate | 0.1 g |
| | Succinic acid | 0.009 g |
| Sweetening: | Liquid sugar | 800 g |
| Vitamin C | | 10 g |

(continued)

| | |
|---|---|
| DRA-containing olive extract (DRA content: 8.9%) | 1 g |
| Vitamin E | 30 g |
| Cyclodextrin | 5 g |
| Flavoring | 15 mL |
| Potassium chloride | 1 g |
| Magnesium sulfate | 0.5 g |

The above-mentioned components were mixed with water to give a volume of 10 L. This drinkable preparation is given at a single dose of about 100 mL.

INDUSTRIAL APPLICABILITY

According to the present invention, there are provided foods and beverages that exhibit high antioxidative activity in the living body for a long time.

## Claims

1. An olive extract comprising desrhamnosyl acteoside in an amount of 0.1% by weight or greater.

2. The olive extract according to claim 1, comprising desrhamnosyl acteoside in an amount of 1% by weight or greater.

3. A composition comprising the olive extract according to claim 1 or 2.

4. The composition according to claim 3, wherein the composition is a food or beverage.

5. A food or beverage comprising the olive extract according to claim 1 or 2.

6. A method for preparing a desrhamnosyl acteoside-containing composition, the method comprising the step of treating an acteoside-containing composition with a glycosidase having rhamnosidase activity.

7. The method according to claim 6, wherein the acteoside-containing composition is an olive extract.

8. The method according to claim 6 or 7, wherein the glycosidase is naringinase.

## Patentansprüche

1. Ein Olivenextrakt, umfassend Desrhamnosylacteosid in einer Menge von 0,1 Gew.-% oder mehr.

2. Der Olivenextrakt nach Anspruch 1, umfassend Desrhamnosylacteosid in einer Menge von 1 Gew.-% oder mehr.

3. Eine Zusammensetzung, umfassend den Olivenextrakt nach Anspruch 1 oder 2.

4. Die Zusammensetzung nach Anspruch 3, wobei die Zusammensetzung ein Lebensmittel oder Getränk ist.

5. Ein Lebensmittel oder Getränk, umfassend den Olivenextrakt nach Anspruch 1 oder 2.

6. Ein Verfahren zur Herstellung einer Desrhamnosylacteosid enthaltenden Zusammensetzung, wobei das Verfahren den Schritt des Behandelns einer Acteosid enthaltenden Zusammensetzung mit einer Glycosidase mit Rhamnosidase-Aktivität umfasst.

7. Das Verfahren nach Anspruch 6, wobei die Acteosid enthaltende Zusammensetzung ein Olivenextrakt ist.

8. Das Verfahren nach Anspruch 6 oder 7, wobei die Glycosidase Naringinase ist.

## Revendications

1. Extrait d'olive comprenant du desrhamnosyl-actéoside en une quantité de 0,1 % en poids ou plus.

2. Extrait d'olive selon la revendication 1, comprenant du desrhamnosyl-actéoside en une quantité de 1 % en poids ou plus.

3. Composition comprenant l'extrait d'olive selon la revendication 1 ou 2.

4. Composition selon la revendication 3, la composition étant un aliment ou une boisson.

5. Aliment ou boisson comprenant l'extrait d'olive selon la revendication 1 ou 2.

6. Procédé de préparation d'une composition contenant du desrhamnosyl-actéoside, le procédé comprenant l'étape de traitement d'une composition contenant de l'actéoside avec une glycosidase ayant une activité rhamnosidase.

7. Procédé selon la revendication 6, dans lequel la composition contenant de l'actéoside est un extrait d'olive.

8. Procédé selon la revendication 6 ou 7, dans lequel la glycosidase est la naringinase.

FIG. 1

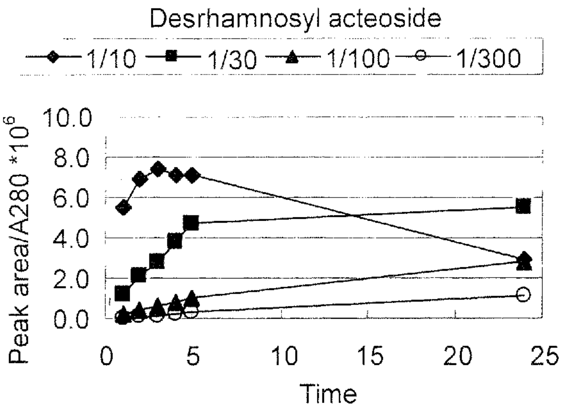


FIG. 2

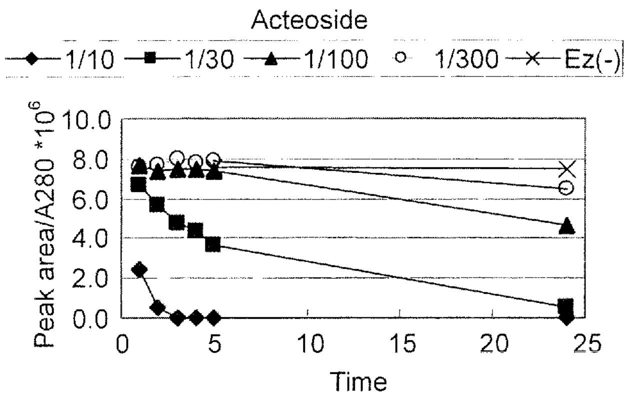


FIG. 3

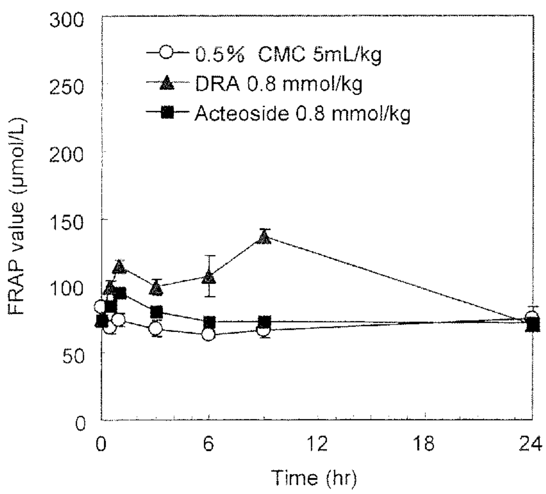

FIG. 4

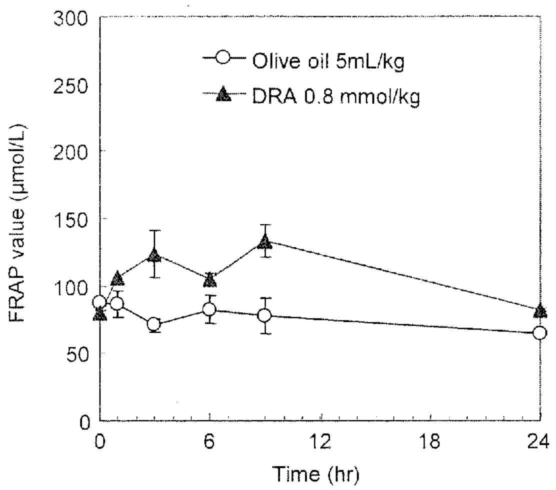


FIG. 5

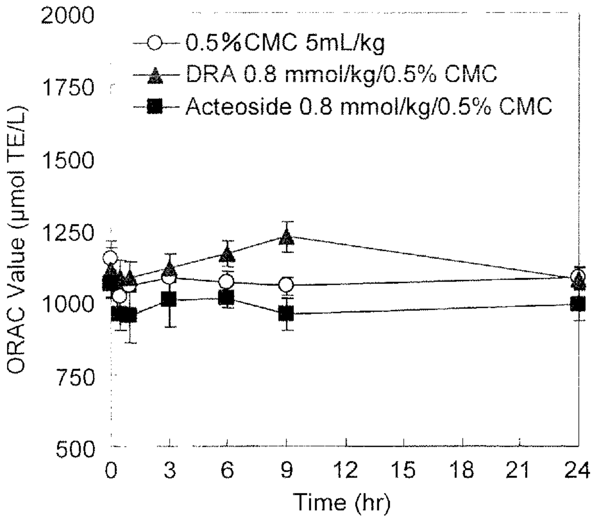


FIG. 6

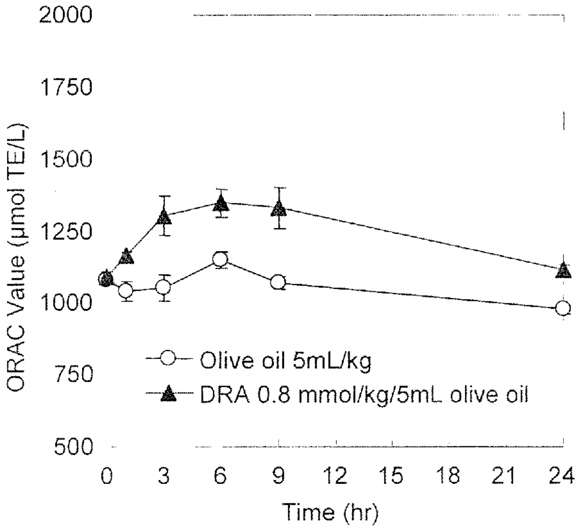

FIG. 7

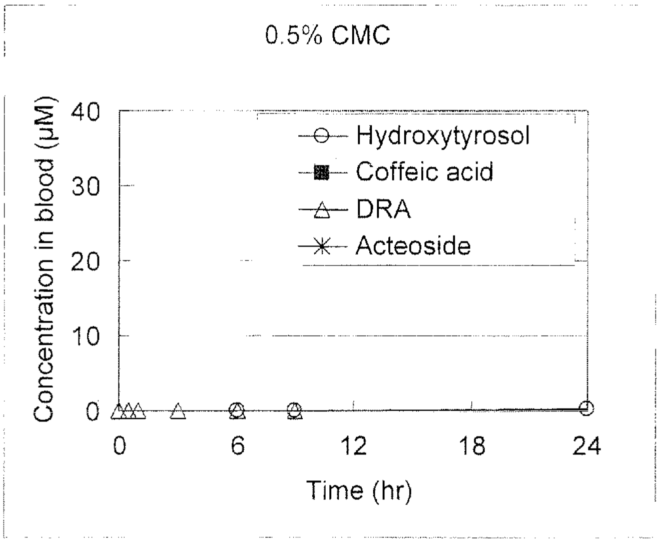


FIG. 8

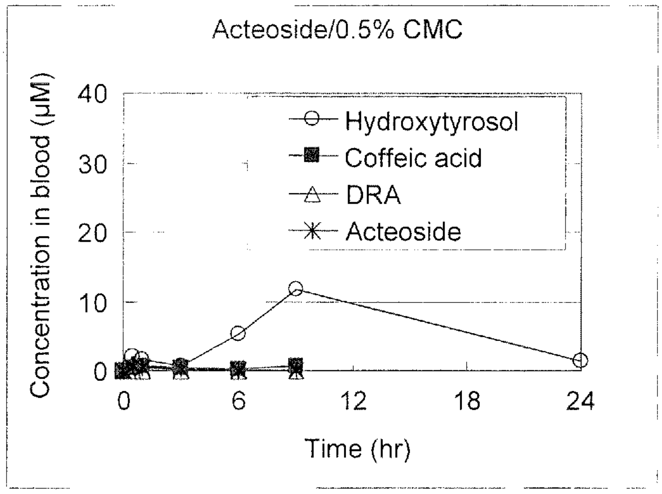


FIG. 9

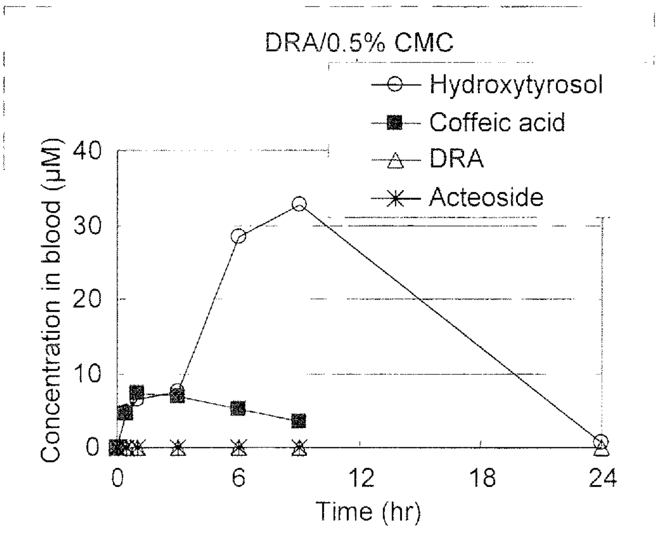

FIG. 10

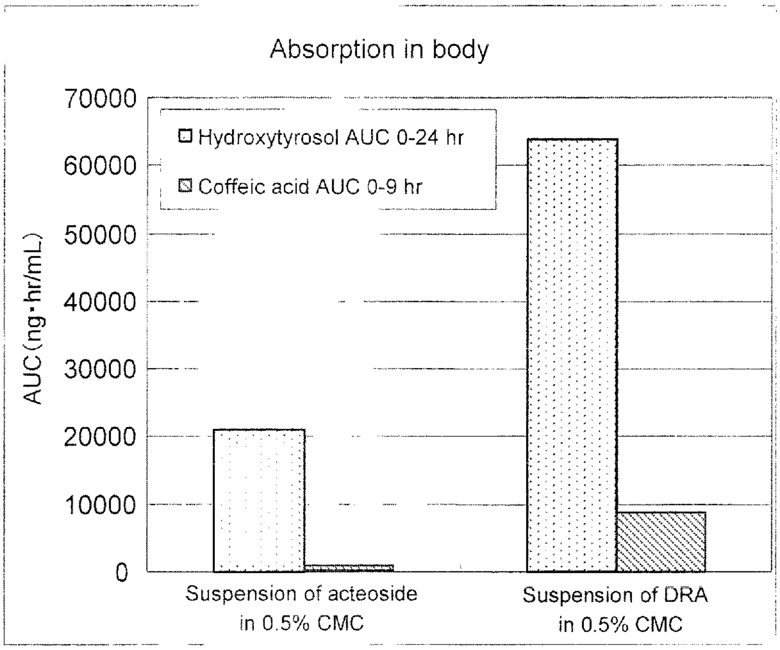
Absorption in body
70000
60000
50000
40000
30000
20000
10000
0
AUC (ng·hr/mL)
Hydroxytyrosol AUC 0-24 hr
Coffeic acid AUC 0-9 hr
Suspension of acteoside in 0.5% CMC
Suspension of DRA in 0.5% CMC

FIG. 11

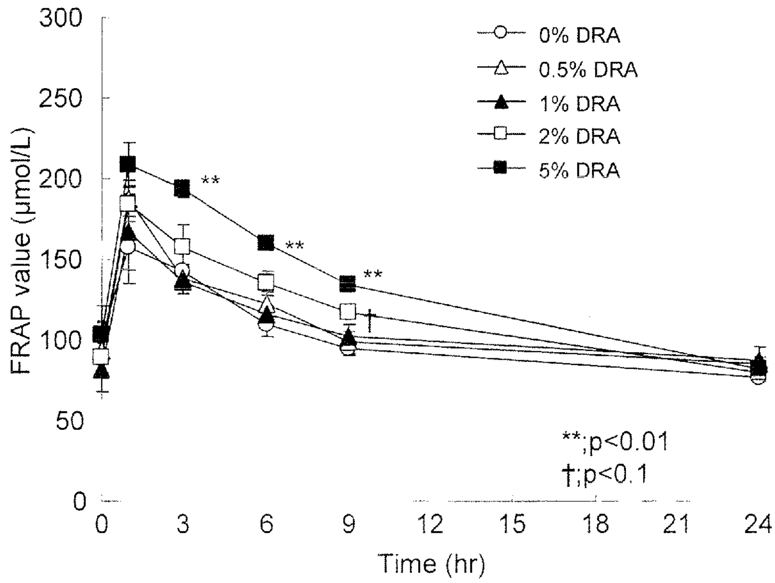
300
250
200
150
100
50
0
FRAP value (μmol/L)
0% DRA
0.5% DRA
1% DRA
2% DRA
5% DRA
**
**
**
†
**;p<0.01
†;p<0.1
0
3
6
9
12
15
18
21
24
Time (hr)

FIG. 12

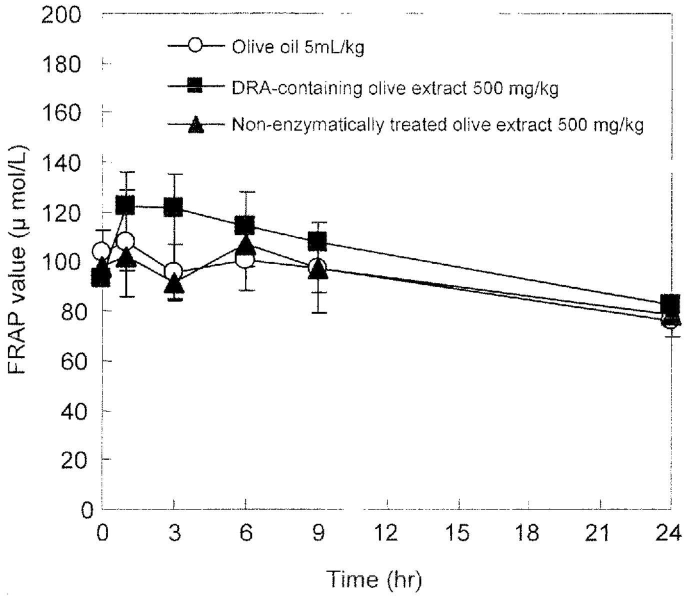
Olive oil 5mL/kg
DRA-containing olive extract 500 mg/kg
Non-enzymatically treated olive extract 500 mg/kg
FRAP value (μ mol/L)
200
180
160
140
120
100
80
60
40
20
0
0
3
6
9
12
15
18
21
24
Time (hr)

FIG. 13

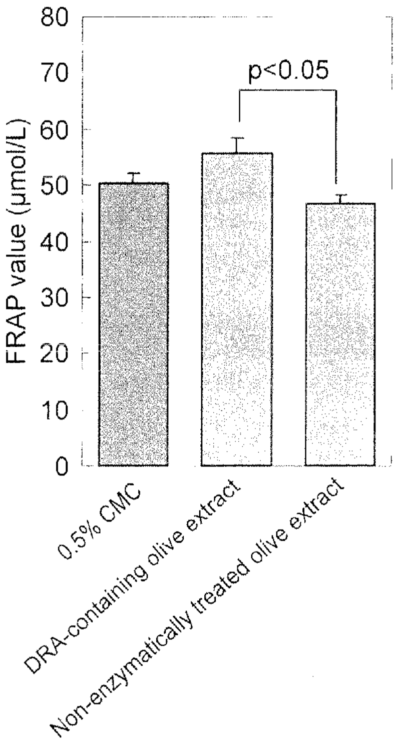
FRAP value (μmol/L)
80
70
60
50
40
30
20
10
0
p<0.05
0.5% CMC
DRA-containing olive extract
Non-enzymatically treated olive extract

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005334022 A
- JP 2002186453 A


### Non-patent literature cited in the description


- *J. Agric Food Chem,* 2004, vol. 52, 479-484
- *J. Agric Food Chem,* 2005, vol. 53, 8963-8969
- *Atherosclerosis,* 2006, vol. 188 (1), 35-42
- *Clin Nutr,* 2006, vol. 25 (5), 859-868
- *Gazzetta Chimica Italiana,* 1986, vol. 116, 431-433
- *Planta Medica,* 2004, vol. 70 (1), 50-53
- *Phytomedicine,* 2007, vol. 14, 659-667
- *J. Chromatogr. B,* 2006, vol. 844 (1), 89-95